(19) Europäisches Patentamt
European Patent Office
Office européen des brevets

(11) EP 1 034 837 B1

(12) **EUROPÄISCHE PATENTSCHRIFT**

(45) Veröffentlichungstag und Bekanntmachung des Hinweises auf die Patenterteilung:
**07.04.2004 Patentblatt 2004/15**

(51) Int Cl.[7]: **B01J 8/18**, B01J 8/24, C07C 17/156, C07C 19/045

(21) Anmeldenummer: **00101325.9**

(22) Anmeldetag: **22.01.2000**

(54) **Verfahren zur Herstellung von Vinylchloridmonomer (VCM)**

Process for producing Vinyl chloride monomere (VCM)

Procédé pour produire le chlorure de vinyle monomère

(84) Benannte Vertragsstaaten:
**BE DE FR GB IT NL**

(30) Priorität: **12.03.1999 DE 19911078**

(43) Veröffentlichungstag der Anmeldung:
**13.09.2000 Patentblatt 2000/37**

(73) Patentinhaber:
- **Uhde GmbH**
  **44141 Dortmund (DE)**
- **Vinnolit Technologie GmbH & Co. KG**
  **84504 Burgkirchen (DE)**

(72) Erfinder:
- **Benje, Michael**
  **64289 Darmstadt (DE)**
- **Schubotz, Thomas**
  **65812 Bad Soden (DE)**
- **Schön, Hartmut**
  **61440 Oberursel (DE)**

(74) Vertreter: **Meinke, Dabringhaus und Partner GbR, Patentanwälte**
**Postfach 10 46 45**
**44046 Dortmund (DE)**

(56) Entgegenhaltungen:
**WO-A-96/26003**
**US-A- 3 679 373**
**US-A- 4 377 491**
**US-A- 2 931 711**
**US-A- 4 197 418**

EP 1 034 837 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Monomeres Vinylchlorid VCM wird bevorzugt aus Ethylen $C_2H_4$ und Chlor $Cl_2$ derart hergestellt, daß hinsichtlich des bei den Umsetzungen erzeugten und verbrauchten Chlorwasserstoffes HCl eine ausgewogene Bilanz erreicht wird.

Das Verfahren zur Herstellung von VCM mit ausgewogener HCl-Bilanz - im folgenden kurz "ausgewogenes VCM-Verfahren" genannt -, besitzt:

- eine Direktchlorierung, in der aus Ethylen $C_2H_4$ und Chlor $Cl_2$ der eine Teil des benötigten Zwischenproduktes 1,2-Dichlorethan EDC erzeugt wird,
- eine Oxichlorierung, in der aus Ethylen, Chlorwasserstoff HCl und Sauerstoff $O_2$ der andere Teil des benötigten Zwischenproduktes 1,2-Dichlorethan EDC erzeugt wird,
- eine EDC-Reinigung, in der beide Teile des EDC-Zwischenproduktes zusammen mit dem aus der Fraktionierung rezirkulierten Rück-EDC von den in der Oxichlorierung bzw. von den in der EDC-Pyrolyse gebildeten Nebenprodukten befreit werden, um ein für den Einsatz in der Pyrolyse geeignetes sogenanntes Spalt-EDC zu gewinnen;
- eine EDC-Pyrolyse, in der das Spalt-EDC thermisch gespalten wird und als Ausbeute im wesentlichen im Spaltgas die Stoffe VCM, HCl und nichtumgesetztes EDC anfallen, jedoch auch noch zusätzliche Begleitstoffe im Spaltgas enthalten sind;
- eine Fraktionierung, in der das als Produkt gewünschte Rein-VCM aus dem Spaltgas abgetrennt und die anderen wesentlichen Spaltgasbestandteile HCl und nichtumgesetztes EDC als Wertstoffe gesondert zurückgewonnen und als wiederverwertbarer Einsatz als Rück-HCl bzw. Rück-EDC im ausgewogenen VCM-Verfahren rezirkuliert werden.

In dem ausgewogenen VCM-Verfahren geschieht vorzugsweise die Oxichlorierung des Ethylen großtechnisch in einer katalytisch wirkenden Wirbelschicht. In dem Zeitschriftenbeitrag [1] wird erläutert, daß die Reaktionstemperatur nicht über 325°C ansteigen darf, weil sonst u.a. auch die Nebenproduktbildung überhand nehmen könnte.

Die Oxichlorierung des Ethylen ist eine ausgeprägt exotherme Reaktion. Wegen der zu begrenzenden Temperaturführung der Reaktion ist die unmittelbare Abfuhr der erzeugten Reaktionswärme zwingend. Die Wirbelschicht wird daher üblicherweise mit geeigneten, der Wirbelschicht wirkungsvoll wärmeentziehenden Oberflächen in Kontakt gebracht.

Die wärmeentziehenden Oberflächen und die anderen vom Wirbelschichtgut beaufschlagten Oberflächen unterliegen dem Verschleiß durch den Korund des Wirbelschichtgutes. Auch sind diese Oberflächen korrosionsgefährdet, da als einer der gasförmigen Reaktanden in der Wirbelschicht der heiße Chlorwasserstoff auf den Oberflächen ansteht.

Aus GB-PS 1 100 439 (Harpring et al.) ist bekannt, daß für die Oxichlorierung des Ethylen in der Wirbelschicht ein Kupfer-Katalysator auf einem Korund-($Al_2O_3$) Träger eingesetzt wird. Das in der Wirbelschicht eingesetzte Katalysator-Schüttgut besitzt einen Cu-Gehalt von etwa 10 Gew.%.

In der NL-PS 65/06985 wird angegeben, daß das katalytisch aktive Kupferimpregnat auf Mikro-Korundkugeln aufgetragen wird, wodurch ein Cu-Gehalt von 10 Gew.% auf den Mikro-Korundkugeln eingestellt wird. Die Mikro-Korundkugeln besitzen Partikelgrößen zwischen 20 µm und 200 µm.

Aus der US-PS 3,488,398 (Harpring et al) ist auch ersichtlich, daß das katalytisch aktive Kupferimpregnat in Form von Kupferhaliden, vorzugsweise jedoch das Kupferchlorid, verwendet wird. Es ist angegeben, daß ein Kupfergehalt im Bereich von 3 Gew.% Cu bis zu 12 Gew.% Cu praktikabel ist, jedoch daß auch Kupfergehalte oberhalb von 12 Gew.% Cu angewendet werden könnten, dies jedoch unzweckmäßig sei, da die Reaktionausbeute dadurch nicht gesteigert werden könne und das katalytisch wirkende Wirbelschichtgut vermehrt dazu neige, im Oxichlorierungsreaktor Anbackungen zu bilden.

Es sind daher in der Zwischenzeit seit dem Aufkommen des ausgewogenen VCM-Verfahrens eine Anzahl von Vorschlägen bekannt geworden, wie in der Oxichlorierungsreaktion die Erzeugung nachteiliger Nebenprodukte möglichst vermieden und zugleich der Reaktor betriebssicher und ohne Verstopfungen erzeugende Katalysatoranbakkungen betrieben werden kann und wobei zugleich auch der betriebsbedingte Abrieb und/oder die betriebsbedingte Korrosion auf ein erträgliches Maß beschränkt bleibt.

In der PCT-Offenlegungsschrift WO 96/26003 (Krumböck) wird ein Oxichlorierungsreaktor mitgeteilt, bei dem ein Reaktandengasstrom entgegengesetzt zum nach oben gerichteten Fluidisierungsgastrom in das Wirbelbett eingespeist wird. Mit dieser Ausführungsform der Gasführung im Wirbelbett soll vornehmlich bewirkt werden, daß der Abrieb nicht mehr oder nur in wesentlich verringertem Umfange auftritt.

Betreiber von VCM-Anlagen, deren Anlagen nach dem ausgewogenen VCM-Verfahren arbeiten, wünschen sich, daß diese Anlagen so gestaltet sind, daß mit ihnen auch über längere Zeiträume unter weitgehender Vermeidung betriebsbedingter und auch störungsbedingter Stillstände produziert werden kann. Für den Oxichlorierungsreaktor

wird gefordert, daß er eine über längere Betriebsdauer stabile Temperaturführung in der Wirbelschicht gestattet, die auch dann noch gewährleistet sein soll, wenn nach einiger Betriebsdauer das aus Mikro-Korundkugeln bestehende Wirbelschichtgut infolge Abriebs eine Verschiebung seines Korngrößenspektrums zu niedrigeren Werten hin erfährt.

Ein frisch eingesetztes Wirbelschichtgut liegt mit mittleren Mikro-Kugeldurchmessern von 50 bis 60μm vor. Durch den im betrieblichen Gebrauch erfolgenden Abrieb stellt sich nach und nach eine mittlere Partikelgröße des Wirbelschichtgutes von 30 bis 40μm ein.

Aufgabe der Erfindung ist daher, ein ausgewogenes Verfahren zur Herstellung von VCM anzugeben.

Mit einem Verfahren zur Herstellung von VCM, wobei in der Oxichlorierung die gasförmigen Reaktanden zusammen mit inertem Trägergas in eine adiabate Reaktionszone eingespeist werden und jene Gase zusammen mit dem in der adiabaten Reaktionszone gebildeten Teil der gasförmigen Reaktionsprodukte in eine oberhalb der adiabaten Reaktionszone angeordnete gekühlte Reaktionszone übergeleitet werden, in der der restliche Umsatz der Reaktanden erfolgt, wobei beide Reaktionszonen einer integralen Wirbelschicht zugeordnet sind, welche von den Gasen fluidisiert wird und in der die Reaktion vom Wirbelschichtgut katalysiert wird und das Reaktionsprodukt und das Trägergas gasförmig oben aus der Wirbelschicht abgezogen werden, und wobei die gekühlte Reaktionszone vollständig von einem senkrechtachsigen, gassenbildenden Kühlstabbündel durchsetzt ist, die Kühlstäbe untereinander im wesentlichen äquidistante Abstände besitzen, der in der gekühlten Reaktionszone angeordnete Teil des Wirbelbettes den wärmeabführenden Oberflächen der Kühlstäbe im wesentlichen vollräumig ausgesetzt ist und in den Gassen zwischen den wärmeabführenden Oberfläche der Kühlstäbe das fluidisierte Wirbelgut und die Gase wärmeabgebend geführt sind, wird diese Aufgabe dadurch gelöst, daß das Verhältnis zwischen dem den Gassenquerschnitt kennzeichnenden äquivalenten Durchmesser zum mittleren Gasblasendurchmesser der Wirbelschicht Werte von 1 bis 6 einschließlich besitzt.

Die Kühlstäbe können im Kühlstabbündel sowohl in rechteckiger als auch in dreieckiger, 60°-Teilung angeordnet sein.

Ein vom Wirbelschichtgut und von den Gasen beaufschlagter Gassenquerschnitt im Kühlstabbündel wird von den Systemlinien der Teilung umschlossen. In diesem von den Systemlinien umschriebenen Quadrat bzw. Dreieck sind auch noch die Querschnitte der Kühlstäbe gelegen. Der vom Wirbelschichtgut und den Gasen durchsetzte freie Querschnitt einer Gasse besitzt eine vieleckig berandete Fläche.

Um die Fläche eines solchen Vielecks analog zur Kreisfläche mit einer einzigen Maßzahl kennzeichnen zu können - bei der Kreisfläche ist diese Maßzahl der Kreisdurchmesser -, wird hier an dieser Stelle eine hinsichtlich der Fläche äquivalente Maßzahl, nämlich der sogenannte "äquivalente Durchmesser" definiert. Der äquivalente Durchmesser wird als Quotient zwischen dem Vierfachen der freien Gassenquerschnittsfläche und dem Umfang der freien Fläche gebildet.

Der mittlere Gasblasendurchmesser der Wirbelschicht wird aus den verfahrenstechnischen Größen, die die Eigenschaften der Wirbelschicht bestimmen, auf rechnerischem Wege ermittelt. Hierzu werden die in dem von J. Werther verfaßten und in der Enzyklopädie [3] veröffentlichten Beitrag genannten Modelle und Beziehungen verwendet.

Im folgenden werden Gleichungen beschrieben, mit denen der äquivalente Blasendurchmesser bestimmbar ist.

A) Blasenwachstum und -zerfall können in Feinkornwirbelschichten (Geldart-Gruppe A) durch folgende Differentialgleichung [5] beschrieben werden:

$$\frac{d}{dh}d_v = \underbrace{\left(\frac{3\epsilon_b}{9\pi}\right)^{1/3}}_{\text{Blasen-wachstum}} - \underbrace{\frac{d_v}{3\lambda u_b}}_{\text{Zerfallsmechanismus}} \qquad (1)$$

| | |
|---|---|
| $\frac{d}{dh}d_v$ = | Änderung des Blasendurchmessers mit der Wirbelschichthöhe (Für Geldart-Gruppe A = O, d.h. Blasendurchmesser = konstant) |
| $d_v$ = | Blasendurchmesser [m] |
| $\varepsilon_b$ = | Blasenvolumenanteil in der Wirbelschicht |
| $\lambda$ = | mittlere Blasenlebensdauer zwischen zwei Zerfallsvorgängen |

B) Lambda (mittlere Blasenlebensdauer zwischen zwei Zerfallsvorgängen) wird nach [5] mit

$$\lambda \approx 280^{*}\frac{u_{mf}}{g} \qquad (2)$$

abgeschätzt. Dabei ist $u_{mf}$ [m/s] die Minimalfluidisierungsgeschwindigkeit und g [9,81 $m/s^2$] die Erdbeschleunigung.

C) Die Reynoldszahl und damit auch die Gasgeschwindigkeit bei Minimalfluidisierung wurde hier nach Wen und Yu [6] abgeschätzt:

$$Re_{mf}=[33,7^{2}+0,0408Ar]^{0,5}-33,7 \qquad (3)$$

(Ar = Archimedeszahl für den jeweiligen mittleren Teilchendurchmesser in der Wirbelschicht)

$$Ar = \frac{g \cdot d_p^{\;3}}{\nu} \qquad \frac{\rho_s \cdot \rho_f}{\rho_f}$$

$d_p$ = Teilchendurchmesser in m
$\zeta_s$ = Katalysatordichte 3.100 $kg/m^3$
$\zeta_f$ = Gasdichte 4,3 $kg/m^3$
$\nu$ = Kin. viskosität des Gases 5,4 $10^{-6}$ $m^2/s$

Für $d_p$ = 38,3 μm ; 35,3 μm ; 40,3 μm (in laufenden Reaktoren gemessen) ergibt sich $u_{mf}$= 0,0012 m/s ; 0,001 m/s; 0,0013 m/s

D) Der Blasenvolumenanteil der Wirbelschicht wird berechnet nach [5]

$$\varepsilon_b = \frac{V_b}{u_b} \qquad (4)$$

Wobei

$$V_b \approx 0,8(u-u_{mf})$$

u = überlagerte Gasgeschwindigkeit = Gesamtgasvolumen/Freier Reaktorquerschnitt [m/s] hier: 0,369 m/s
$V_b$ = Gasblasenvolumenstrom (ausgedrückt als Geschwindigkeit bezogen auf den freien Reaktorquerschnitt)
$u_{mf}$ = Gasgeschwindigkeit bei Minimalfluidisierung

und

$$u_b=V_b+0,71^{*}\vartheta^{*}\sqrt{g^{*}d_v} \qquad [m/s] \qquad (5)$$

$u_b$ = Aufstiegsgeschwindigkeit der Blasen

Für Feinkornwirbelschichten (Geldart-Gruppe A) gilt:

$$\vartheta=3.2d_t^{0,33};0,05\leq d_t\leq 1m \qquad (6)$$

$d_t$ ist der hydraulische Durchmesser des Reaktors [m], gebildet mit dem Reaktorinnendurchmesser und den kumulierten Oberflächen des Kühlstabbündels.

Beispiel: $d_t$ (60°-Teilung) = 0.24m; $d_t$ (90°-Teilung) = 0.38m.

Aus den obigen Gleichungen läßt sich der Gleichgewichtsblasendurchmesser iterativ bestimmen.

Dazu wird als Fig. 1 ein erfindungsgemäß gestalteter Oxichlorierungsreaktor dargestellt. Es ist der Längsschnitt durch den Apparat gezeigt. In der unteren Hälfte des Reaktors ist als Zentrum der Bereich der gekühlten Reaktionszone durch eine schraffierte Bezugsfläche bezeichnet. In diesem Bereich sind im Reaktorinneren durch gestrichelte Linien die Mittelachsen der Kühlstäbe des Kühlstabbündels angedeutet.

Unterhalb der gekühlten Reaktionszone ist die adiabate Reaktionszone im Reaktor angeordnet. Sie liegt oberhalb des mit den Einströmdüsen bestückten, gewölbten Reaktorbodens und unterhalb der unteren horizontalen Begrenzungsebene des Kühlstabbündels.

Wird der Reaktor betrieben, so durchsetzt die Wirbelschicht beide Zonen.

Im oberen Teil des Reaktors sind die Feinstaubabscheidung und Feinstaubrückführrohre angeordnet. Damit wird die Feinstaubfraktion des Wirbelbettgutes vor dem Austrag zusammen mit den am oberen Ende des Reaktors austretenden Gasen bewahrt.

Fig. 2 zeigt einen linken, halben Reaktorquerschnitt mit den in 90°-Teilung angeordneten rohrförmigen Kühlstäben des Kühlstabbündels. Fig. 2a zeigt ein Feld 90°T der verwendeten 90°-Teilung.

Fig. 3 zeigt einen rechten, halben Reaktorquerschnitt mit den in 60°-Teilung angeordneten rohrförmigen Kühlstäben des Kühlstabbündels. Fig. 3a zeigt ein Feld 60°-T der verwendeten 60°-Teilung.

Der Rohrdurchmesser der Kühlstäbe in Fig. 2 ist größer als der in Fig. 3.

Im folgenden werden die zu beiden beispielgebenden Ausgestaltungen der vorliegenden Erfindung gehörenden Abmessungen und verfahrenstechnischen Größen gegenübergestellt.

Es versteht sich von selbst, daß in der Erfindung die Beaufschlagung des Kühlstabbündels mit Wärmeträgermedium nicht auf Wärmeträgeröl beschränkt ist. Gleichermaßen ist die Strömungsführung des Wärmeträgermediums weder auf eine ausschließliche Hintereinanderschaltung der einzelnen Kühlstäbe noch auf eine ausschließliche Parallelschaltung der einzelne Kühlstäbe im Bündel beschränkt, es können auch Mischformen eingesetzt werden, z. B. die partielle Serienschaltung mindestens zweier Kühlstäbe zu einer Gruppe, die dann als zu mehreren Gruppen parallelgeschaltet mit Wärmeträgermedium beaufschlagt werden. Solche Anordnungen werden z.B. bei Wärmeaustauschern als sogenannte Wechselfeldberohrung bezeichnet.

Auch versteht es sich von selbst, daß die Erfindung nicht auf die beiden beispielgebenden Ausführungen beschränkt ist, wobei in der nun folgenden Aufstellung der Abmessungen und verfahrenstechnischen Größen in der ersten der linken Spalte die Daten der 90°-Teilung und in der zweiten, der rechten Spalte die Daten der 60°-Teilung genannt sind.

| | 90°-Teilung | 60°Teilung |
|---|---|---|
| Kühlrohraußendurchmesser | 114,3 mm | 88,9 mm |
| Rohrwandabstand im Kühlstabbündel | 88.9 mm | 63 mm |
| $\lambda$ | 0.034 s | 0.034 s |
| $\mathbf{u}_b$ | 1,18 m/s | 1,06 m/s |
| $\varepsilon_b$ | 0,23 | 0,28 |
| $\mathbf{d}_v$ | 0,031 m | 0,03 m |

Man erkennt, daß eine unterschiedliche Kühlrohrgeometrie und damit ein unterschiedlicher Durchmesser des Reaktors die Blasengröße nur unwesentlich beeinflußt.

Der Vergleich des Wärmeübergangs bei konstruktiv unterschiedlicher Asuführung des Kühlstabbündels zeigt überraschenderweise, daß die vorliegende Erfindung ein breites Spektrum der konstruktiven Gestaltung der Reaktoren abdeckt.

Mittels einer von Kunii und Levenspiel vorgeschlagenen Berechnungsgleichung [7] läßt sich für das Verhältnis des für die 60°-Teilung zu erwartenden Wärmeübergangskoeffizienten zum Wärmeübergangskoeffizienten der 90°-Teilung schreiben:

$$\frac{\alpha_{bw,60}}{\alpha_{bw,90}} = \sqrt{\frac{f_{w,60}(1-\varepsilon_{bw,60})}{f_{w,90}(1-\varepsilon_{bw,90})}} \qquad (7)$$

$\alpha_{bw}$ = Wärmeübergangskoeffizient Rohrwand / Fließbett [$W/m^2K$]

$f_w$ = Blasenfrequenz [1/s]

$\varepsilon_{bw}$ = Blasenvolumenanteil an der Rohroberfläche

Dabei setzt man voraus, daß die Wärmeleitfähigkeit der Emulsionsphase und die Suspensionsphasenporösität unverändert bleiben. Da die Blasendurchmesser klein gegen den Durchmesser des Reaktors sind, kann man annehmen, daß die Blasen zufällig über den Reaktorquerschnitt verteilt aufsteigen. In diesem Fall können die an der Wärmetauscheroberfläche geltenden Werte $f_w$ und $\varepsilon_{bw}$ durch die lokale Blasenfrequenz f und den lokalen Blasenvolumenanteil $\varepsilon_b$ ersetzt werden:

$$\frac{\alpha_{bw,60}}{\alpha_{bw,90}} = \sqrt{\frac{f_{,60}(1-\varepsilon_{b,60})}{f_{,90}(1-\varepsilon_{b,90})}} \quad (8)$$

Für die Blasenfrequenz läßt sich schreiben:

$$f = \frac{3}{2}\,\frac{u_b \varepsilon_b}{d_v} \quad (9)$$

Gleichung (9) verknüpft die lokale Blasenfrequenz mit der lokalen Blasengröße, der Steiggeschwindigkeit und dem Blasenvolumenanteil. Einsetzen von Gleichung (9) in Gleichung (8) liefert:

$$\frac{\alpha_{bw,60}}{\alpha_{bw,90}} = \sqrt{\left(\frac{u_{b,60}}{u_{b,90}}\right)\cdot\left(\frac{d_{v,90}}{d_{v,60}}\right)\cdot\left(\frac{\epsilon_{b,60}\,(1-\epsilon_{b,60})}{\epsilon_{b,90}\,(1-\epsilon_{b,90})}\right)} \quad (10)$$

Mit den hier rechnerisch ermittelten Werten, jeweils gerechnet für die Mitte des Wärmetauscherbündels und

| | 90°-Teilung | 60°-Teilung |
|---|---|---|
| $u_b$ | 1,18 m/s | 1,06 m/s |
| $\varepsilon_b$ | 0,23 | 0,28 |
| $d_v$ | 0,031 m | 0,03 m |

ergibt sich

$$\alpha_{bw,60} / \alpha_{bw,90} = 1.03$$

d.h. der Wärmeübergangskoeffizient einer 60°-Teilung wird sogar geringfügig besser werden als der einer 90°-Teilung. Hieraus wird ersichtlich, daß die vorliegende Erfindung es möglich macht, sowohl eine enge, platzsparende Teilung des Kühlstabbündels als auch eine vergleichsweise großzügigere Teilung des Kühlstabbündels vorzusehen.

Dem Gestalter der Reaktoreinbauten steht dadurch ein von mehreren Parametern definiertes Kennfeld zur Verfügung, innerhalb dessen er eine optimale Auswahl der Katalysatoreigenschaften je nach Bedarf mehr auf eine geringere Backneigung bzw. mehr auf geringerem Abrieb bzw. mehr auf den geringeren Katalysator-Aktivitätsverlust hin ausgerichtet vornehmen kann. Dies ist als ein wesentlicher und überraschender Vorzug der Erfindung anzusehen.

## Verzeichnis der zitierten Veröffentlichungen

[1] McPherson, R. W. et al., "Vinyl Chloride Monomer ... What You Should Know", Hydrocarbon Processing, 58. Jahrgang, März 1979, S. 75-88

[2] J.S. Nyworski, E.S. Velez, "Oxychlorination of Ethylene", Applied Industrial Catalyses, Vol. I, 1983, Academie Press Inc., ISBN: 0-12-440201-1, S. 239-273

[3] "Ullmanns Enzyklopädie der technischen Chemie", 4. Auflage (1975), Band 9, S. 448, Abb. 16

[4] D. Kunii und O. Levenspiel, "Fluidization Engineering", Butterworth-Heinemann 1991, S. 329

[5] "Ullmann's Encyclopedia of Industrial Chemistry", VCH, Weinheim, 1992, Vol. B4, S. 248

[6] C.Y. Wen und Y.H. Yu, AICHE J. 4 (1958), S. 15

[7] D. Kunii und O. Levenspiel, "Fluidization Engineering", Butterworth-Heinemann 1991, S. 329

**Patentansprüche**

1. Verfahren zur Herstellung von VCM, wobei in der Oxichlorierung die gasförmigen Reaktanden zusammen mit inertem Trägergas in eine adiabate Reaktionszone eingespeist werden und jene Gase zusammen mit dem in der adiabaten Reaktionszone gebildeten Teil der gasförmigen Reaktionsprodukte in eine oberhalb der adiabaten Reaktionszone angeordnete gekühlte Reaktionszone übergeleitet werden, in der der restliche Umsatz der Reaktanden erfolgt, wobei beide Reaktionszonen einer integralen Wirbelschicht zugeordnet sind, welche von den Gasen fluidisiert wird und in der die Reaktion vom Wirbelschichtgut katalysiert wird und das Reaktionsprodukt und das Trägergas gasförmig oben aus der Wirbelschicht abgezogen werden, und wobei die gekühlte Reaktionszone vollständig von einem senkrechtachsigen, gassenbildenden Kühlstabbündel durchsetzt ist, die Kühlstäbe untereinander im wesentlichen äquidistante Abstände besitzen, der in der gekühlten Reaktionszone angeordnete Teil des Wirbelbettes den wärmeabführenden Oberflächen der Kühlstäbe im wesentlichen vollräumig ausgesetzt ist und in den Gassen zwischen den wärmeabführenden Oberflächen der Kühlstäbe das fluidisierte Wirbelgut und die Gase wärmeabgebend geführt sind,
**dadurch gekennzeichnet,**
**daß** das Verhältnis zwischen dem den Gassenquerschnitt kennzeichnenden äquivalenten Durchmesser zum mittleren Gasblasendurchmesser der Wirbelschicht Werte von 1 bis 6 einschließlich besitzt.

2. Verfahren nach Anspruch 1,
**dadurch gekennzeichnet,**
**daß** die Kühlstäbe im Kühlstabbündel in rechteckiger 90°- und/oder dreieckiger 60°-Teilung angeordnet sind.

3. Verfahren nach Anspruch 1 oder 2,
**dadurch gekennzeichnet,**
**daß** als Wärmeträgermedium Wärmeträgeröl und von diesem Wärmeträgeröl durchströmte Rohrschlangen eingesetzt werden, deren Wandabstand zueinander und/oder zur Reaktorwand wenigstens dem mittleren Gleichgewichtsblasendurchmesser des das Katalysatorbett durchströmenden Gases entspricht.

4. Verfahren nach einem der vorangehenden Ansprüche,
**dadurch gekennzeichnet,**
**daß** die Wärmeabfuhr im Reaktor durch fühlbare Wärme erfolgt.

5. Verfahren nach einem der vorangehenden Ansprüche,
**dadurch gekennzeichnet,**
**daß** ein gegen Zusammenbacken oder Anbacken resistentes Katalysatorgranulat eingesetzt wird.

6. Anlage zur Durchführung des Verfahrens nach einem der vorangehenden Ansprüche,
**dadurch gekennzeichnet,**
**daß** bei Einsatz eines kupferhaltigen Katalysators zur Umsetzung von Ethylen, Sauerstoff und HCl zu 1,2-Dichlorethan und Wasser der Außendurchmesser der eingesetzten Kühlrohre im Bereich von 25 bis 100 mm und der Rohrwandabstand im Bereich von 30 bis 90 mm liegt.

7. Anlage zur Durchführung des Verfahrens nach einem der vorangehenden Ansprüche,
**dadurch gekennzeichnet,**
**daß** die dünneren Kühlrohre in Dreiecksteilung im Reaktor angeordnet sind.

8. Anlage zur Durchführung des Verfahrens nach einem der vorangehenden Ansprüche,

**dadurch gekennzeichnet,**
**daß** die Anzahl der Rohre pro Rohrschlange zwischen 5 und 20 liegt.

## Claims

1. Method for the production of VCM, wherein in the oxychlorination, the gaseous reactants together with inert carrier gas are fed into an adiabatic reaction zone, and those gases together with that part of the gaseous reaction products that is formed in the adiabatic reaction zone are conveyed into a cooled reaction zone that is arranged above the adiabatic reaction zone, in which the remaining transformation of the reactants takes place, wherein both reaction zones are assigned to an integral fluidised bed which is fluidised by the gases and in which the reaction is catalysed by the fluidised bed material[2] and the reaction product and the carrier gas are drawn off in a gaseous form at the top from the fluidised bed, and wherein the cooled reaction zone is completely pervaded by a vertical-axis, channel-forming bundle of cooling rods, the cooling rods being essentially equidistant from one another, that part of the fluidised bed that is arranged in the cooled reaction zone being exposed, essentially over the whole space, to the heat-sinking surfaces of the cooling rods, and the fluidised fluidised-bed material and the gases are led in a heat-emitting manner in the channels between the heat-sinking surfaces of the cooling rods,
**characterised in that**
the ratio between the equivalent diameter that characterises the channel cross section to the mean gas bubble diameter of the fluidised bed has values of 1 to 6 inclusive.

2. Method in accordance with claim 1, **characterised in that** the cooling rods are arranged in the cooling bundle in a rectangular 90° and/or triangular 60° division.

3. Method in accordance with claim I or 2, **characterised in that** heat transfer oil, and pipe coils through which this heat transfer oil flows, are used as a heat transfer medium, the wall distance [between the pipe coils] relative to one another and/or to the reactor wall corresponding to at least the mean equilibrium bubble diameter of the gas flowing through the catalyst bed.

4. Method in accordance with one of the preceding claims, **characterised in that** the heat dissipation in the reactor takes place through sensible heat.

5. Method in accordance with one of the preceding claims, **characterised in that** a catalyst granulate is used that is resistant to agglomeration or baking on.

6. Plant for carrying out the method in accordance with one of the preceding claims,
**characterised in that**
where a catalyst which contains copper is used for transforming ethylene, oxygen and HCl into 1,2-dichloroethane and water, the outer diameter of the cooling pipes used lies in the range from 25 to 100 mm and the distance between pipe walls lies in the range from 30 to 90 mm.

7. Plant for carrying out the method in accordance with one of the preceding claims, **characterised in that** the thinner cooling pipes are arranged in a triangular division in the reactor.

8. Plant for carrying out the method in accordance with one of the preceding claims, **characterised in that** the number of pipes per pipe coil is between 5 and 20.

## Revendications

1. Procédé pour la production de MCV tel que, dans l'oxychloration, les réactants gazeux sont introduits conjointement avec un gaz support inerte dans une zone de réaction adiabatique et ces gaz-là sont transférés, conjointement à la partie des produits de réaction gazeux formée dans la zone de réaction adiabatique, dans une zone de réaction refroidie placée au-dessus de la zone de réaction adiabatique, zone de réaction refroidie dans laquelle se produit le reste de la conversion des réactants, les deux zones de réaction étant associées à une couche fluidisée intégrale qui est fluidisée par les gaz et dans laquelle la réaction à partir du produit de la couche fluidisée est catalysée et le produit de réaction et le gaz support sont soutirés sous forme de gaz en haut, de la couche fluidisée et la zone de réaction refroidie étant entièrement traversée par un faisceau de barres de refroidissement à axe perpendiculaire formant des voies, les barres de refroidissement étant essentiellement équidistantes l'une par rapport à l'autre, la partie du lit fluidisé placée dans la zone de réaction refroidie étant exposée essentiellement dans la totalité de son volume aux surfaces dissipatrices de chaleur des barres de refroidissement et le produit fluidisé et les gaz étant conduits avec cession de chaleur dans les voies entre les surfaces dissipatrices de chaleur des barres de refroidissement,
**caractérisé en ce que**,
le rapport entre le diamètre équivalent caractérisant la section des voies et le diamètre moyen des bulles de gaz de la couche fluidisée est compris entre 1 et 6 inclus.

**2.** Procédé selon revendication 1,
**caractérisé en ce que**
les barres de refroidissement sont placées dans le faisceau de barres de refroidissement en segmentation rectangulaire de 90° et/ou triangulaire de 60°.

**3.** Procédé selon revendication 1 ou 2,
**caractérisé en ce que**
on utilise comme fluide caloporteur une huile caloporteuse et des serpentins traversés par cette huile caloporteuse, dont la distance entre paroi de l'un par rapport à l'autre et/ou par rapport à la paroi du réacteur correspond au moins au diamètre moyen des bulles à l'équilibre du gaz traversant le lit catalyseur.

**4.** Procédé selon l'une des revendications précédentes,
**caractérisé en ce que**
la dissipation de chaleur dans le réacteur se déroule par chaleur sensible.

**5.** Procédé selon l'une des revendications précédentes,
**caractérisé en ce que**
on utilise un granulat catalyseur résistant à l'aglomération ou à l'encrassement.

**6.** Installation pour la réalisation du procédé selon l'une des revendications précédentes,
**caractérisé en ce que**
le diamètre extérieur des tubes réfrigérants utilisés est compris entre 25 et 100 mm et la distance entre paroi des tubes entre 30 et 90 mm si l'on utilise un catalyseur contenant du cuivre pour la conversion d'éthylène, d'oxygène et de HCl en 1,2-dichloréthane et eau.

**7.** Installation pour la réalisation du procédé selon l'une des revendications précédentes,
**caractérisé en ce que**
les tubes réfrigérants plus minces sont placés en segmentation triangulaire dans le réacteur.

**8.** Installation pour la réalisation du procédé selon l'une des revendications précédentes,
**caractérisé en ce que**
le nombre de tubes par serpentin est compris entre 5 et 20.

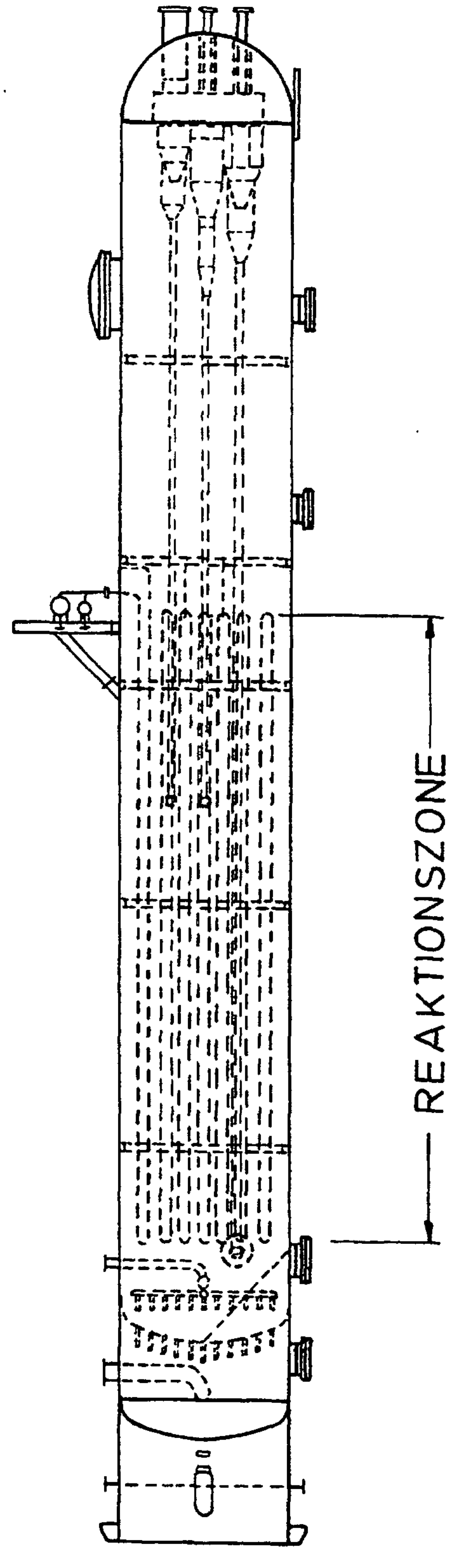


FIG. 1

FIG. 2

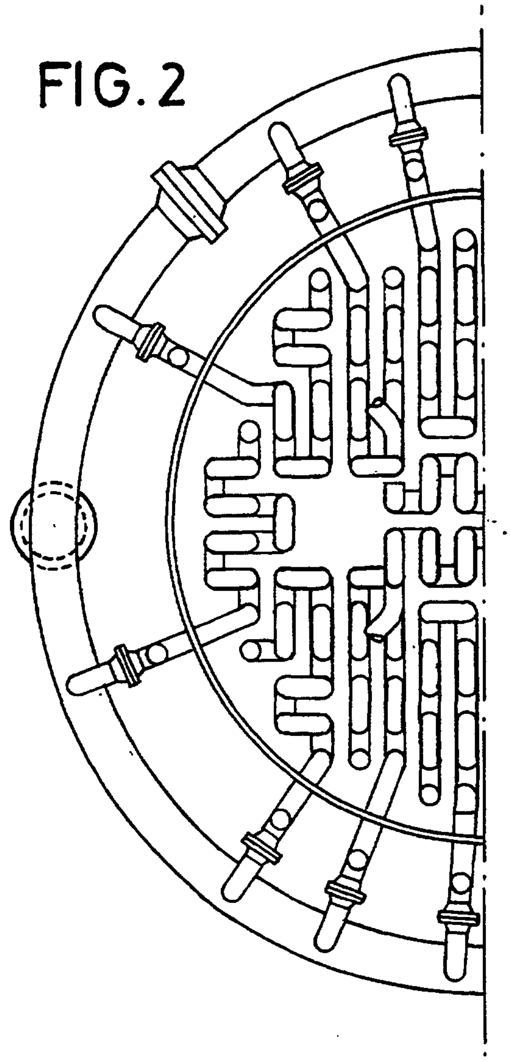

FIG. 3

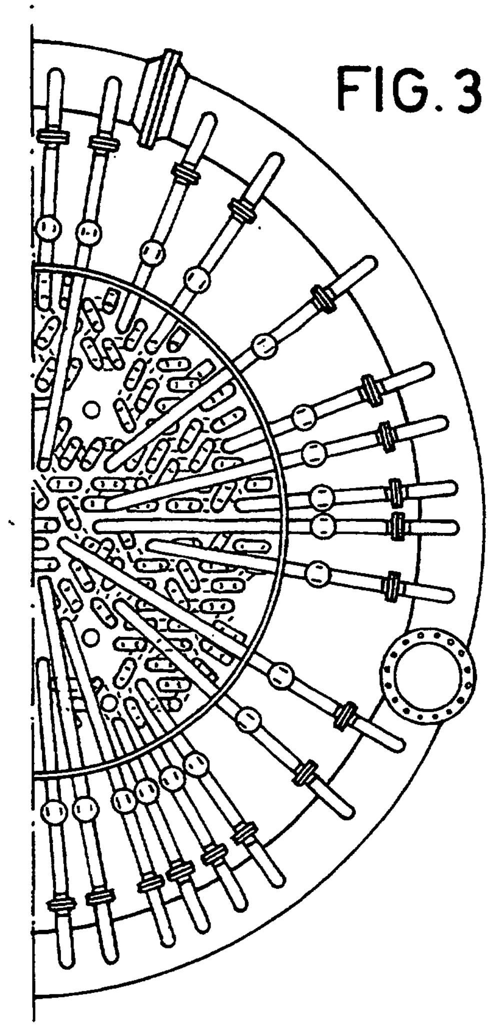

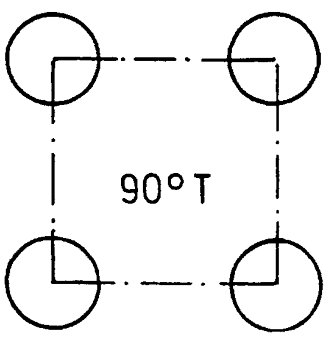


FIG. 2a

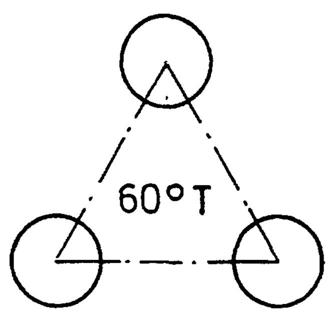


FIG. 3a